Europäisches Patentamt

European Patent Office

Office européen des brevets

(11) Publication number: **0 123 928**
A2

# EUROPEAN PATENT APPLICATION

(21) Application number: 84103551.2

(22) Date of filing: 30.03.84

(51) Int. Cl.³: **C 12 N 15/00**, C 12 N 9/52, C 12 N 9/64, C 12 N 1/20 // (C12N1/20, C12R1/19),(C12N9/52, C12R1/19)

(30) Priority: 31.03.83 US 480860

(43) Date of publication of application: 07.11.84 Bulletin 84/45

(84) Designated Contracting States: **AT BE CH DE FR GB IT LI LU NL SE**

(71) Applicant: **CODON GENETIC ENGINEERING LABORATORIES, 430 Valley Drive, Brisbane California 94005 (US)**

(72) Inventor: **Cashion, Linda M., 650 Poirier Street, Oakland, Cal., 94609 (US)**
Inventor: **McCaman, Michael T., 746 Cherry Avenue, San Bruno, Cal. 94066 (US)**
Inventor: **Rice, Craig W., 2000 Crystal Springs Rd, San Bruno, Cal. 94066 (US)**
Inventor: **Sias, Stacey R., 37 Carlson Ct., San Anselmo, Cal. 94960 (US)**

(74) Representative: **Reinländer & Bernhardt Patentanwälte, Orthstrasse 12, D-8000 München 60 (DE)**

(54) Recombinant DNA coding for a polypeptide displaying milk clotting activity.

(57) Methods and compositions are provided for synthesizing within a bacterial host a polypeptide which displays milk clotting activity. Recombinant DNA material derived from bovine rennin, living cells containing such material and methods for producing and using such material, together with the polypeptide, are provided to clot milk with high efficiency.

```
P = Pst
B = Bam HI
T = Taq
```

EP 0 123 928 A2

## Description
## Recombinant DNA Coding for a Polypeptide Displaying Milk Clotting Activity

Technical Field

This invention relates to the clotting of milk, used as a step in the process of making of cheese. More specifically the invention relates to the synthesis using recombinant DNA techniques of a polypeptide derived from calf rennin which displays milk clotting activity.

With the advent of recombinant DNA technology, it is now possible to introduce foreign genes into microorganisms and regulate the level of their expression.

The numerous proteins which make up living organisms are the expression products of the information contained in deoxyribonucleic acid (DNA). This information is coded by the order of the nucleic acid bases on the linear DNA sequence. The four bases, adenine (A), thymine (T), cytosine (C) and guanine (G) are arranged in a linear sequence as a single chain of DNA and each triplet of bases, called a codon, defines a single amino acid. Because there are 64 possible combinations of bases and only 20 amino acids, some amino acids may be defined by multiple codons. Three of the codons specify non-amino acid signals.

The sequence of codons which directs the synthesis of a single polypeptide is known as a structural gene. Regulatory sequences on either side of the structural gene control the level of polypeptide synthesized in a cell.

The information contained in the DNA is transcribed into an intermediate, called messenger ribonucleic acid (mRNA), wherein the DNA base T is replaced by a distinct RNA base uracil (U). This mRNA is then translated into a polypeptide by the action of a ribosome.

Gene expression is modulated by the aforementioned regulatory DNA sequences in both transcription to the RNA and translation to the protein. Transcription of DNA into mRNA is controlled by promoter sequences adjacent to the structural gene.

Following the initiation of transcription, ribosomes bind to a specific sequence near the beginning of the mRNA transcript. Translation is initiated at a start signal codon, which is essentially always a methionine codon (AUG). Thus for the efficient transcription and translation of the nucleic acid codon it is necessary to have a structural gene, a promoter region, a ribosome binding site, and a translational initiator.

To prepare recombinant DNA which contains the above elements and will express foreign genes in a host cell, one first purifies mRNA from tissues which express the desired polypeptide. The structural gene DNA may be reconstructed from the mRNA sequence by the enzyme reverse transcriptase, which has been isolated from an avian retrovirus. This complementary DNA (cDNA) can then be joined to bacterial DNA using the enzyme DNA ligase, isolated from bacteriophage T4. The bacterial DNA used in this ligation is known as the cloning vector and is usually an extrachromosomal DNA sequence which replicates autonomously, called a plasmid. This autonomous replication is advantageous because with appropriate manipulation the cell can be induced to replicate numerous copies of the recombinant plasmid and thus preferentially express the desired products. Furthermore plasmids usually contain structural genes that confer resistance to antibiotics and other growth inhibitors and thus cells which contain

0123928

the complete plasmids may be detected through appropriate screening techniques. The reconstructed recombinant plasmid may be introduced directly into the host cell by a process known as transformation.

In order to obtain large amounts of the recombinant DNA, the plasmid DNA may be induced to replicate to very high copy numbers within the host cell by a process known as amplification. The inserted eukaryotic DNA can then be removed from the cloning vector DNA by digestion with restriction endonucleases, which cleave the DNA at precisely defined sequences. This cleavage usually forms cohesive termini which can be easily religated.

Expression of the eukaryotic DNA within foreign cells is effected by the insertion of a strong promoter sequence and ribosome binding site adjacent to the start of the structural gene. The mRNA structural genes are generally "read" by the cellular machinery in only one direction. The reading proceeds from the 5' -end towards the 3'-end and this defines the "stream" of the RNA. Control sequences such as promoters and ribosome binding sites are positioned "upstream" from the structural gene. These control sequences are derived from normal host cell genes which can be regulated with appropriate bacterial cofactors. An efficient expression vector can be constructed using host cell regulatory sequences and a selectable plasmid gene which confers resistance to an appropriate growth inhibitor. Thus host cells which contain the complete recombinant plasmid may be detected and levels of protein synthesis from the cloned gene can be specifically augmented by treatment with the appropriate cofactor.

Once the eukaryotic gene has been expressed in the host cell, the polypeptide must be purified and, in many cases, enzyme activity must be recovered.

0123928

Rennin, also known as chymosin (EC3.4.23.4), is the active component of rennet which is used to clot milk in the process of making cheese. Commercial preparations of calf rennet are obtained from the mucosal lining of the fourth stomach of unweaned calves (the abomasum). A reduction in the current veal market and subsequent increase in cheese consumption has forced the dairy industry to find substitutes for calf rennet. In fact there is only enough calf rennet to supply 20% of the cheese market.

Proteinases isolated from fungi have been employed in conjunction with or as a substitute for bovine rennet. See for example U.S. Patent Nos. 3,151,039 and 3,212,905. However, these aspartate proteinases have a broader substrate range and are more stable during the milk clotting process. This increase in proteolytic activity and stability can alter the flavor and texture of the final cheese product. Moreover, losses of protein during cheese making are often greater with rennet substitutes, causing a lower yield. In order to provide the dairy industry with an enzyme preparation which is a polypeptide derived from authentic bovine rennet and purified from a non-animal source, recombinant DNA techniques have been utilized.

Bovine rennin is synthesized in two active forms, chymosin-A and chymosin-B, which differ only in a single amino acid at position #290. Rennin is synthesized as a precursor which contains a signal peptide of 16 amino acids. Harris, T.J.R., et al., Nucl. Acids Res. 10:2177-87 (1982); Moir, D., et al., Gene, 19:127-38 (1982). This signal peptide is cleaved as the protein is secreted from the cell. Once secreted the zymogen, called prorennin, undergoes activation under the acidic conditions of the stomach to generate mature rennin. The activation is apparently autocatalytic and may occur in stages. (B. Foltmann, Trav. Lab. Carlsberg, 35:143-231 (1966)). Within prorennin two peptide bonds can be cleaved autocatalytically, the Phe-Leu bond between amino acids 28 and 29, and the Phe-Gly bond between amino acids 42 and 43. In vitro studies have indicated that at pH 2 the first bond is preferentially cleaved to generate pseudorennin, while at pH 4.7 the second bond is cleaved to generate mature rennin. Both forms of rennin are active in clotting milk.

Attempts have been made to produce authentic rennin by recombinant DNA means. See, e.g. Alford, B.L. et al., European Patent Application Serial No. 82100124.5 filed January 8, 1982. However, the methods require complicated construction strategies to complete the rennin structural gene, and suffer from low efficiency of expression. Thus it is an object of this invention to provide an active polypeptide which can be used by the dairy industry as a substitute for bovine rennin.

It is a further object of this invention to provide living cells containing recombinant DNA material which synthesizes with high efficiency a polypeptide having milk clotting activity.

It is a still further object of this invention to provide alternative recombinant DNA which may be adapted to particular host cell cultures.

It is another object of this invention to provide DNA sequences derived from bovine rennin which code for polypeptides having milk clotting activity.

It is another object of this invention to provide methods for synthesis of polypeptides specified by DNA sequences derived from bovine rennin in a bacterial cell.

It is another object of this invention to provide methods for recovery of polypeptides which are synthesized in bacterial cells containing DNA sequences derived from bovine rennin and which are able to clot milk.

It is still another object of this invention to provide methods of constructing recombinant DNA incorporating DNA sequences derived from bovine rennin.

It is yet another object of this invention to provide methods of clotting milk using polypeptides having milk clotting activity which are synthesized from DNA sequences derived from bovine rennin.

Disclosure of the Invention

In accordance with this invention, methods and compositions are provided for synthesizing within a bacterial host a polypeptide which displays milk clotting activity. According to a method of this invention, recombinant DNA may be constructed which includes a structural gene which codes for a polypeptide displaying milk clotting activity. In this method a DNA sequence which codes for said polypeptide is derived from bovine sources, and inserted into a cloning vector with a promoter and ribosomal binding site upstream from the DNA sequence. This recombinant DNA is then used to transform a bacterial host and the host is cultured under conditions which promote expression of the polypeptide.

Also in accordance with this invention, compositions are provided which are capable of synthesizing a polypeptide displaying milk clotting activity. Using the methods and compositions provided in this invention, a polypeptide is obtained which displays milk clotting activity and which may be synthesized at high levels of efficiency in a host cell system.

Brief Description of the Drawings

Figure 1 displays the restriction enzyme maps of two DNA fragments obtained by reverse transcriptase treatment of bovine mRNA and which code for a polypeptide displaying milk clotting activity.

Figure 2 shows the nucleotide sequence of one of the RNA fragments mapped in Figure 1. Figure 2 also shows the corresponding amino acid sequence for which this RNA fragment codes.

Figure 3 schematically shows the method used in section 8 for inserting a DNA fragment as described in Figure 2 into an appropriate expression vector derived from plasmid pBR322.

Figure 4 shows the DNA nucleotide sequence coding for a fusion peptide and including the promoter sequence, amino acid codons and rennin-derived codons through codon 42. Figure 4 also shows the corresponding amino acid sequence where appropriate.

Best Mode For Carrying Out The Invention

The present invention provides a methods and compositions to generate a polypeptide having milk clotting activity, using recombinant DNA technology. Sequences coding for bovine rennin were isolated from stomach tissue, transcribed into cDNA, and cloned into a bacterial cloning vector. The nucleotide sequences were then transferred into specially designed expression plasmids in order to maximize synthesis of the polypeptide. After purification, in vitro conditions of the calf stomach were duplicated to acid activate the synthetic rennin polypeptide.

Stomachs from unweaned calves were selected for mRNA isolation because this tissue produces in vivo the highest levels of active rennin. Reverse transcriptase was used to transcribe mRNA into a cDNA copy to be cloned in bacteria. However, reverse transcriptase is prone to premature termination of transcription; hence, a size selection was carried out to enrich for full length gene copies. Two clones were analyzed in detail. One contained the entire coding region for the A form of rennin, while the other contained all the sequences of the mature A form of rennin but lacked sequences from the N-terminal cleavage fragment of prorennin. The second clone also contains sequences from the 3'-untranslated region proceeding the poly-A tail.

With these two rennin clones, the DNA sequences were rearranged in order to maximize their expression. The cloned DNA fragments are like "cassettes" that can be inserted into a variety of specially constructed expression vectors. It is necessary to build into a efficient expression vector a strong promoter for RNA polymerase and an effective ribosome binding site to initiate protein synthesis. With the use of specific restriction endonucleases, we were able to insert the DNA sequence into a bacterial expression plasmid so that the structural gene for calf rennin can be transcribed in response to the bacterial promoter.

The polypeptide is synthesized in bacterial cells at high levels. However, the purification of the polypeptide is complicated by its tendency to aggregate and sediment with bacterial membranes. When the polypeptide is purified with bacterial membranes, it is not active. Activity can be restored by extraction of the polypeptide from membrane components with urea. Treatment with urea apparently denatures the polypeptide molecules which can then refold into an active conformation, and enzyme activity can be measured following acid activation.

0123928

## Experimental Details

### 1. Isolation of mRNA

Preparation of Tissue: Rennin is synthesized by the gastric glands located in the mucosal tissue of the fourth stomach (abomasum) of unweaned calves. This tissue was obtained from a local abattoir. The stomachs were removed from freshly slaughtered animals and packed in ice. The abomasum, which is the most prominent stomach in the unweaned calf, was inspected for the presence of "cheese" (to insure the animal was pre-rumminant) and washed with water. The mucosal material was scraped from the stomach and quickly frozen in a dry ice-methanol bath. An average of 80 grams of tissue was obtained from each animal. Since mRNA degrades rapidly, the tissues were frozen immediately after sacrifice of the animal and stored at -70°C until needed.

Isolation of RNA: The mucosal tissue contains high levels of mRNA to direct the synthesis of rennin. Total cellular RNA was extracted from the mucosal tissue, and then the mRNA population was specifically isolated. In order to protect cellular RNA from degradation, the tissue was weighed and then quickly submerged in a solution of guanidinium thiocyanate to inhibit cellular RNAse as described by Ullrich, A. et al., Science 196, 1313-1319 (1977). The tissue was thoroughly mascerated in a Waring blender, and the RNA was isolated by sedimentation through a CsCl cushion by the procedure of V. Glisin, R. Crkvenjakov, and C. Byus, Biochem. 13, 2633-2637 (1974). The total weight of mucosal tissue used as starting material was 13.3 grams. From this, 8.5 milligrams of total cellular RNA was recovered. This yield (.06%) is within the expected range of .01 - 1%.

Purification of mRNA: The mRNA fraction of total cellular RNA differs from other RNA species in that it contains 15-40 adenylic acid residues on the 3'-end of the molecule. The mRNA population was purified by chromatography on oligo-dT cellulose as described by Aviz, J. and Leder, P., Proc. Natl. Acad. USA 69, 1408-1412 (1972). The mRNA molecules anneal to the column by virtue of the polyadenylated tail and can be selectively purified. From the 8.5 milligrams of calf stomach RNA purified by the above procedure 30.8 micrograms of mRNA was recovered. The mRNA population comprises approximately 2% of the total RNA. Our yield of 0.35% is less than 2% and indicates that the non-messenger RNA species have been removed. The mRNA population contains RNA templates for all proteins which were actively synthesized by the unweaned calf abomasum at the time of death. Rennin should comprise 1% - 5% of the total protein in the abomasum. Thus, if the mRNA is 50% pure, 0.5% - 2.5% of the final clones should contain rennin sequences.

2.  Translation of Rennin mRNA in Vitro

Before this mRNA was used as a template for the in vitro DNA synthesis it was essential to verify that molecules coding for preprorennin were present. A cell free extract of rabbit reticulocytes (reticulocyte lysate) will faithfully translate purified mRNA into protein in vitro (Pelham, H. R. B. and Jackson, R. J. Eur. J. Biochem. 67, 247-256, 1976). In this system the abomasum-derived mRNA directed the synthesis of at least twelve distinct polypeptide species which ranged in molecular weight from 17,000 to 102,000 daltons. The bands were discrete and few short peptides were synthesized. The most prominent species had an apparent molecular weight of 38,000-41,000, the approximate size of preprorennin.  This is also the size of prepepsinogen, another enzyme synthesized by the abomasum tissue.  To confirm that the mRNA purified from the abomasum did direct the synthesis of preprorennin, the in vitro translation products were  precipitated with anti-serum raised in rabbits against purified calf rennin.  Only one major band was precipitated by the anti-serum, and the molecular weight was between 38,000-41,000, which is consistent with preprorennin.  Similar translation assays with preprorennin mRNA were also carried out in frog oocytes with the same result.

3.    Identification of Rennin-Specific mRNA Species

In addition to in vitro translation to identify rennin mRNA within the mRNA population from calf abomasum, hybridization of the mRNA with rennin-specific probes was also carried out.  The sequence of the rennin-specific probes was derived from the amino acid sequence published by Foltmann, B., et al., J. Biol. Chem. 254, 8447-8456 (1979).  From the universal genetic code, the probable DNA sequence of a gene or template can be predicted from the amino acid sequence of the protein.  The DNA sequence is only probable because the code is degenerate -- some amino acids are represented by more than one codon.  An analysis of the amino acid sequence of prorennin pointed to two regions containing only amino acids with unique codons. Fortunately, these regions are not sequences common between prorennin and pepsinogen.  In these two small regions the genetic code predicts exactly the mRNA sequence.  Oligomers complementary to these short sequences of genetic information were synthesized for use as probes.  The smaller probe of 12 nucleotides (GTTCATCATGTT) is complementary to the genetic information for amino acids 183-186 of rennin. The second probe contains a mixture of two 14-mers (TACTGCTGTTTCTG and TACTGCTGGTTCTG) and is analogous to amino acids 29-33.

Hybridization to rennin-specific RNA was carried out using the procedure of Shinnick, T.M. et al., Nucl. Acids Res. 2, 1911-29, (1975). The abomasum mRNA was size-fractionated on an agarose gel which was then dried. The synthetic oligomers were radioactively labeled with $^{32}$P to serve as probes for complementary mRNA sequences. The dried gel was incubated with the radioactive probes and subsequently washed. The radioactive probe will remain bound to the gel by hybridization to complementary sequences in the mRNA. Therefore, hybridization to an RNA band within the agarose gel suggests that rennin-specific mRNA was present in a particular size class. These regions of complementarity could be visualized following auto-radiography. A full length preprorennin mRNA would have to be at least 1095 nucleotides in length to contain the codon for all 365 amino acids of the prorennin polypeptide. Both probes hybridized to the same region of the gel representing mRNA of 1350 bases. When mRNA recovered from this region of a parallel gel was translated in vitro, using the rabbit reticulocyte lysate system, it directed the synthesis of polypeptides of molecular weights 38-41,000. Hence, these data provide strong evidence that the mRNA population contained molecules that specifically encode for preprorennin.

4. Synthesis of cDNA

The rennin mRNA preparation was transcribed into cDNA for cloning in bacteria by the action of reverse transcriptase as described by Ullrich, A., et al. supra. Transcription was initiated at the 3'-polyadenylated tail of rennin mRNA using oligo-dT as a primer. Specifically, ten micrograms of oligo-dT was annealed to ten micrograms of purified mRNA in the presence of 50 mM NaCl. The annealing reaction was heated to 90°C and then slowly cooled. For the reverse transcriptase reaction, deoxynucleosidetriphosphates (A,T,G,C) were added to 0.5 mM along with 40 units of enzyme. The reverse transcriptase reaction buffer was as follows: 15 mM Tris-HCl, pH 8.3, 21 mM KCl, 8 mM $MgCl_2$, 0.1 mM EDTA, and 30 mM 2-mercaptoethanol. This mixture was incubated at 42°C for 45 minutes. The RNA-DNA duplex was disrupted by boiling for three minutes.

The second DNA strand was copied using DNA polymerase (The Klenow fragment). Following transcription of the first DNA strand by reverse transcriptase, a hairpin loop is generated at the end of the DNA transcript that can be used to prime the second DNA strand. The reverse transcriptase reaction mixture was diluted 1:1 with Klenow buffer (35 mM KCl, mM Tris-HCl, pH 8.3, 4 mM $MgCl_2$ , and 15 mM 2-mercaptoethanol) and 15 units of purified Klenow fragment was added. The enzyme mixture was incubated at 14°C for 3 hours and then at 4°C for 16 hours. The duplex DNA was precipitated with ethanol at this point. The nuclease $S_1$ was used to render the cDNA molecules blunt-ended and to open up the hairpin loop of the duplex DNA. The precipitated DNA was dissolved in $S_1$ buffer (25 mM sodium acetate, pH 4.5, 300 mM NaCl, and 1mM $ZnCl_2$) and 1000 units of $S_1$ was added. The $S_1$ reaction was incubated for 90 minutes at 37°C. Following this reaction, the DNA was again precipitated with ethanol.

Transcription of mRNA into the first strand of cDNA using reverse transcriptase is very inefficient, and premature termination is frequent. In order to enrich for full length copies cDNA and eliminate the screening of many clones containing short cDNA molecules, the cDNA preparation was subjected to polyacrylamide gel electrophoresis. The region of the gel corresponding to molecular sizes of 1.0 to 1.5 kb of DNA was excised, and the DNA removed by electroelution. From the original ten micrograms of purified mRNA, 300 nanograms of electrophoretically purified cDNA was recovered.

5. Construction of the Rennin cDNA Plasmid

For isolation of rennin-specific DNA, the blunt-ended cDNA molecules were inserted into bacterial plasmids. We utilized the GC tailing method of Chang, A.C.Y. Nature 275, 617-624 (1978) to insert the cDNA molecules into the plasmid pBR322 (Bolivar, F. et al., Gene 2, 95-113, 1977). Polycytidylic acid residues were polymerized onto the ends of the cDNA molecules using the enzyme terminal deoxynucleotidyl transferase, and polyguanidylic acid residues were added to the ends of the linear form of pBR322. The two tailed DNA preparations were annealed to generate recombinate DNA molecules.

In the formation of cDNA plasmids, ten nanograms of purified cDNA was used. The cDNA was added to the terminal transferase reaction containing: 140 mM potassium cacodylate, 25 mM Tris, pH 7.6, 1 mM $CoCl_2$, and 0.2 mM DTT. Since polycytidylic acid residues were polymerized onto the cDNA molecules, dCTP was added to 1.5 mM. This mixture was prewarmed to 37°C and then 15 units terminal transferase was added. The enzyme reaction was stopped after four minutes by the addition of EDTA to 5 mM.

The reaction was extracted with phenol, and the DNA was precipitated with ethanol. The plasmid DNA pBR322 was digested with the restriction enzyme Pst I,

and then polyguanidylic acid residues were polymerized onto ends of the linear molecule as described above.

At this point, the tailed cDNA molecules were recombined with bacterial plasmid DNA by annealing of the complementary ends. The two preparations of dC-tailed cDNA and dG-tailed pBR322 DNA were mixed in annealing buffer (0.1 M NaCl, 10 mM Tris, pH 7.5, and 0.25 mM EDTA) and heated to 80°C. The mixture was allowed to slow cool to 37°C and then to 26°C.

The recombinant DNA molecules were then introduced into bacteria by transformation. The Escherichia coli strain K-12 MM 294 (ATCC accession No. 31446) was used. Other host cells could also be used for this step. The transformed bacteria were inoculated onto agar plates containing the antibiotic tetracycline. Since the plasmid pBR322 contains the tetracycline resistance gene, only those bacteria which have acquired a recombinant plasmid will survive. These bacteria will each grow and divide to form a bacterial colony. Each cell in the colony will be a descendant of the original parental cell and will contain the same recombinant plasmid. From the ten nanograms of cDNA used to make recombinant plasmids, 2000 clones were obtained and further screened for rennin sequences.

6.  Identification of Rennin Clones

The isolated colonies containing recombinant plasmid DNA were screened using the procedures of Grunstein, M., and Hogness, D.S., Proc. Natl. Acad. Sci. USA 72 3961-3965 (1975). Each recombinant clone was inoculated into a numbered well of a microtiter plate and also onto nitrocellulose filters where the bacteria can grow as isolated colonies. The colonies on the filter were lysed, and filter was then washed to remove cell membranes and proteins. The bacterial DNA, including chromosomal and the recombinant plasmid, specifically adhere to the nitrocelluose filter.

The analysis of the genetic content of plasmid DNA depends on the availability of an appropriate probe. Previously it was demonstrated that the 12-mer and 14-mer were specific probes for rennin in that they hybridized to only a single region of the RNA gels corresponding to prorennin mRNA. These probes were therefore used to identify recombinant plasmids which might contain prorennin sequences. (Wallace, R.B. et al., Nucl. Acids Res. 9, 879-894, 1981). The nitrocellulose filters which had been inoculated with bacterial clones containing recombinant plasmids were immersed in a solution of either the radioactively labeled 12-mer or 14-mer. The labeled oligonucleotides will bind to the filter only when they hybridize to complementary sequences in the recombinant DNA. Areas of radioactivity on the filter are identified by autoradiography. In this manner, from the 2000 clones whose recombinant plasmids were screened, 18 were positive by hybridization to the radioactive probes. This result of 0.9% was within the expected range of 0.5-2.5% positive clones predicted from our estimate of the percentage of rennin mRNA in the cells of the abomasum.

7.   Analysis of Clones that Hybridize to
     Rennin-Specific Clones

The cultures corresponding to the positive plasmids were grown for further analysis. Plasmid DNA was purified from all positive rennin clones, and the size of the inserted cDNA determined by digestion with Pst I, followed by gel electrophoresis. Only two clones contained cDNA inserts large enough to contain all of the genetic information for a full length copy of rennin. This was not an unexpected result, since the initial transcription step in the synthesis of cDNA from mRNA is prone to premature termination. The two clones selected for further study were 5G3 which contained an insert of over 1.4 kb and 15C5 with an insert of 1.2 kb.

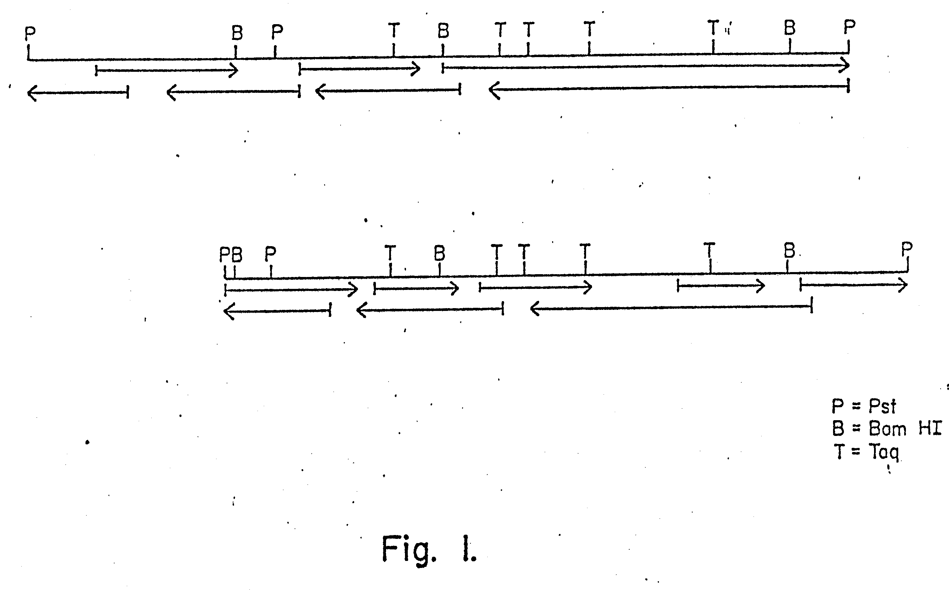

The recombinant plasmids were digested with a variety of restriction endonucleases to determine the position of key restriction sites within the cDNA clones. From this restriction analysis, the two restriction maps presented in Figure 1 were generated. The position of key restriction sites within the cloned material is needed to develop a strategy to sequence the rennin DNA. This sequence analysis was necessary to verify that our clones contain sequences for authentic rennin.

The clones, 5G3 and 15C5, were sequenced by the method of Maxam, A. and Gilbert, W. Proc.Nat. Acad. USA $\underline{74}$, 560-564 (1977). Fragments were generated initially by restriction enzyme digestion of the DNA with either Pst I, Bam HI or Taq I. Other enzymes were also used when necessary. The fragments were labelled with $^{32}$P either by the enzyme T4 polynucleotide kinase or by DNA polymerase I (Klenow fragment) (Maxam and Gilbert, supra).

Both clones were sequenced in their entirety using this method. In addition, 80% of 5G3 and 70% of 15C5 were sequenced on both strands. The homology between the two clones is 100% in the overlapping areas (Figure. 1). Only the clone 5G3 is full length and contains all the sequences encoding preprorennin. In addition, 5G3 contains an additional 250 bases on the 5'end of the rennin structural gene. This region is derived from an inverted repeat of the carboxy region of rennin along with sequences from the untranslated region between the termination codon and the poly A addition site. The clone 15C5 begins at amino acid 6 of prorennin and includes approximately 100 additional nucleotides from the untranslated region on the 3'-end of the structural gene. Both clones encode sequences for the A form of rennin.

Comparison of 5G3 and 15C5 with the published sequences of Harris et al., supra indicated very few differences. The sequence varied at the codons for amino acid 258 and 320, but these were single base differences which did not alter the amino acid. The sequence from Harris et al., supra, also showed a difference at the codon for amino acid 286 that does alter the amino acid to the B form of rennin. The sequence published by Moir et al., supra, is the A form similar to 5G3 and 15C5 and shows no sequence difference. The complete preprorennin sequences of Harris et al., supra, and Moir et al., supra, were derived from sequence data of several overlapping clones. Neither group obtained a full length copy of the preprorennin clone.

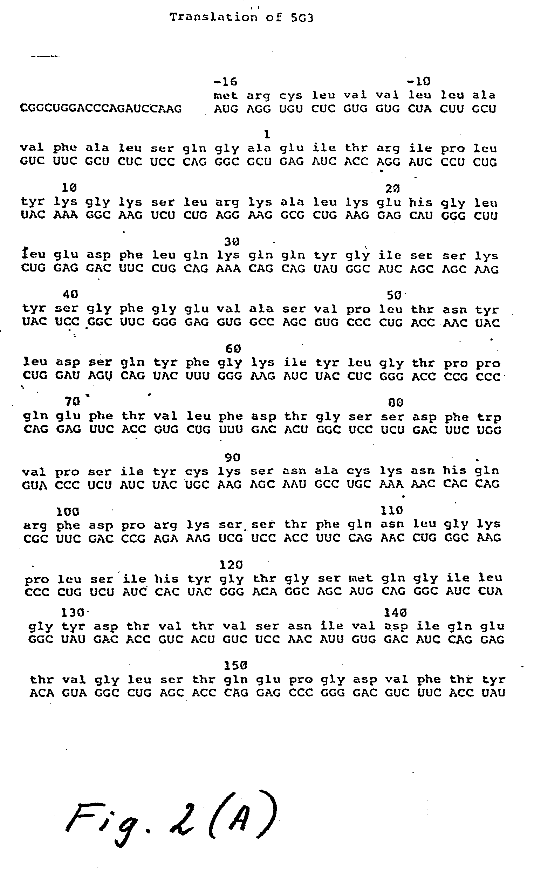

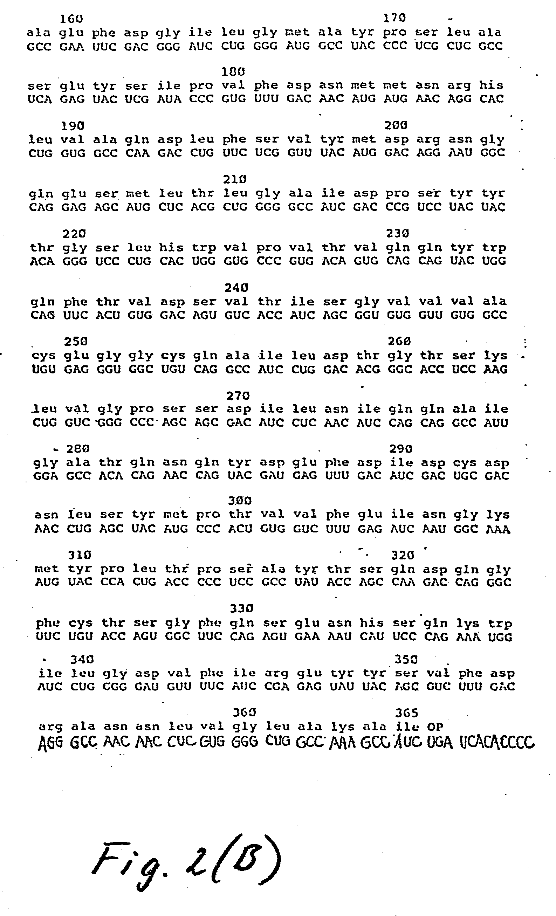

Our predicted amino acid sequence (Fig. 2) agrees with the sequence of the A form of rennin published by Foltmann et al., supra except for the presence of an asparagine codon at amino acid 202 that replaces the aspartic acid. This difference may or may not be significant since a common problem in protein sequence analysis is the difficulty in differentiating between asparagine and aspartic acid.

8.  Construction of Polypeptide Expression Plasmid

Cloned cDNA from eukaryotic sources usually does not contain the proper signals to effect proper transcription and translation in E. coli. Following isolation of appropriate cDNA clones, the eukaryotic DNA must be manipulated so that it can be recognized by the protein synthetic machinery of the host pro- caryotic cell. In order to synthesize derived prorennin-derived polypeptide in E. coli, a recombinant plasmid was constructed that directs the synthesis of a fusion protein between some amino acids derived from a normal bacterial protein, B-galactosidase, an our prorennin-derived polypeptide. Our construction utilized the promotor, ribosome binding site and the several amino acids from the N-terminus of B-galactosidase including the initiator methionine. These sequences were joined in phase via common restriction endonuclease sites with rennin-derived sequences. In the bacterial cell, prorennin-derived polypeptide will be synthesized as a fusion protein but during acid activation of the polypeptide, the bacterial amino acids are cleaved off and the mature polypeptide recovered.

The B-galactosidase sequences used in this construction were derived from the plasmid pUC8 (J. Messing, R. Crea, and P. H. Seeburg. Nucl. Acids Res. 9, 309-321, 1981). In this plasmid, a synthetic oligonucleotide containing convenient restriction endonuclease cleavage sites was inserted near the sequences coding for the N-terminal amino acids of beta-galactosidase. The prorennin-derived sequences were inserted in the synthetic region downsteam from the initiator codon and in phase with this codon.

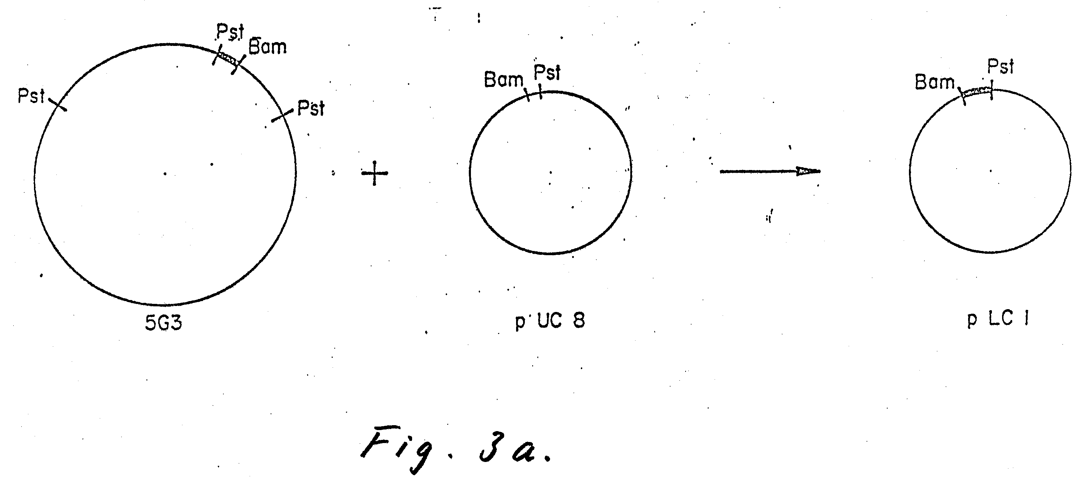

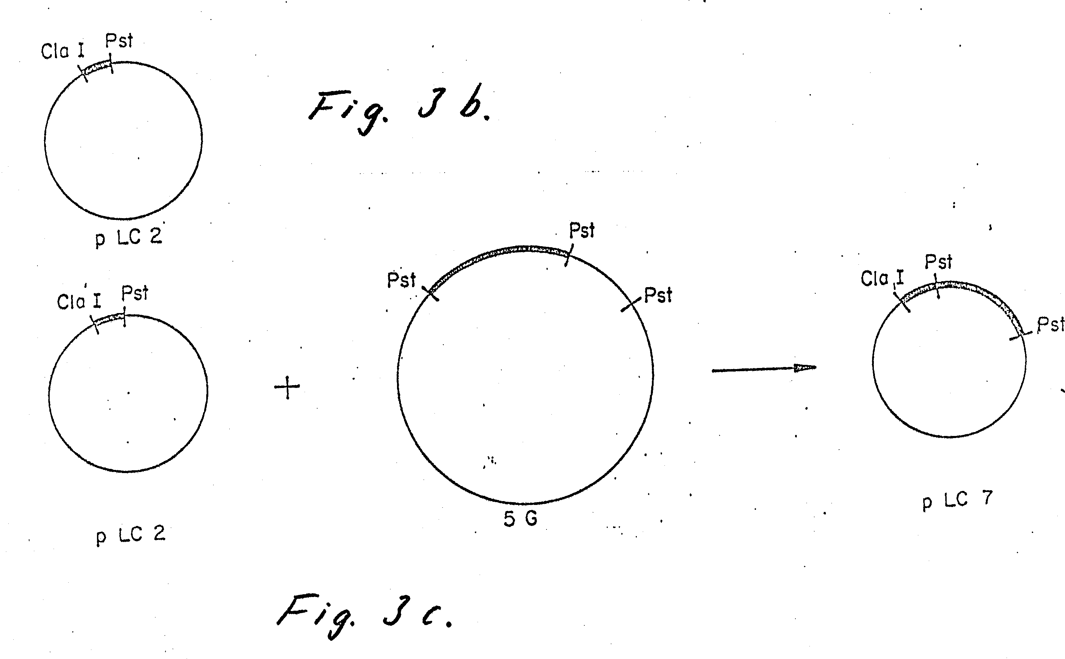

In the first step of this construction, a 70 bp fragment was removed from the cDNA clone 5G3 by digestion with the enzymes Bam HI and Pst I. This 70 bp fragment contained the coding sequences for amino acids 6 through 28 of the prorennin-derived polypeptide. The plasmid pUC8 was also digested with Bam HI and Pst I, and then ligated with the 70 bp fragment via cohesive termini to generate the recombinant plasmid, pLC1 (Fig. 3a). After this ligation step, the prorennin-derived sequences were not in phase with coding sequences of B-galactosidase. In order to change the reading frame, pLC1 was digested with Bam HI to linearize the plasmid, and the overhanging ends were filled-in with the Klenow fragment of DNA polymerase. After ligation, a Cla I restriction site was generated at the site of the former Bam HI site (Fig. 3b).

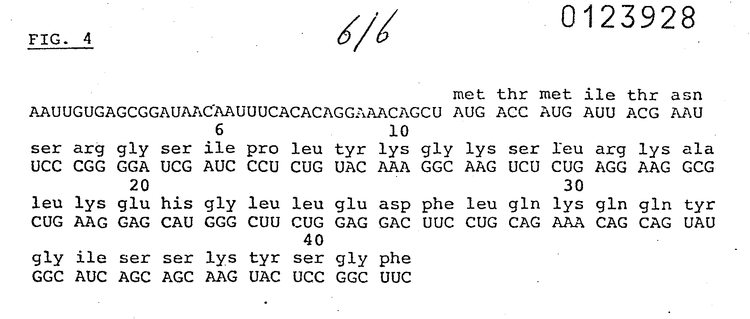

This plasmid (pLC2) was digested with Pst I, followed by treatment with bacterial alkaline phosphatase. The rennin-derived cDNA clone 5G3 was also digested with Pst I, and the 1 kb fragment containing sequences coding for amino acids 29 to the carboxy-terminus of the prorennin-derived polypeptide was isolated. The 1 kb Pst I fragment from 5G3 was ligated into the Pst I linearized plasmid pLC2, and clones were screened for the proper orientation of the 1 kb Pst I fragment. One clone (pLC7) contained the prorennin-derived sequence fused in phase with B-galactosidase (Figs. 3c, 4). The pLC7 prorennin expression plasmid includes sequences which code for both the pseudorennin and mature rennin cleavage sites between amino acids 28-29 and amino acids 42-43, respectively. This clone was used for polypeptide expression and activity assays.

9. Expression and Analysis of Cloned Gene Products

Proteins encoded by the pLC7 DNA were synthesized and identified in three systems: first, by coupled in vitro transcription/translation; second, by radio-labeling minicell gene products; and third, by fractionation of whole E. coli cells. The polypeptides produced in these three systems were electrophoresed on polyacrylamide gels to compare the molecular weights of the plasmid-encoded products with those of authentic prorennin and rennin.

The _in vitro_ transcription/translation system uses the bacterial components necessary for the transcription and translation of plasmid encoded genes in a cell-free environment as described by Zubay, G. Ann. Rev. Gen. 7, 267-287 (1973), and modified by Andrews, W. and Rawson, J. Plasmid 8, 148 (1982). When cloned pLC7 DNA was introduced into this system, radio-labeled protein which co-electrophorese with calf prorennin (M.W. of 41,000) could be detected.

Under acidic conditions, calf prorennin undergoes cleavage to smaller molecular weight forms that demonstrate milk-clotting activity. Efficient acid activation requires the concentration of prorennin to be greater than 5 ug/ml. Therefore, the addition of unlabeled calf prorennin (at a final concentration of 0.1 mg/ml) to the in vitro mixture containing pLC7 polypeptide product was necessary to visualize acid activation. Upon acid activation of a mixture of radiolabeled LC7 polypeptide and calf prorennin, both demonstrated a change in mobility corresponding to conversion of prorennin to pseudorennin (M.W. of 38,000) when activated at pH 2 or to rennin (M.W. of 36,000) when activated at pH 4.5. Thus, the pLC7 encoded polypeptide can be cleaved at the appropriate pH to smaller molecular weight polypeptides which are the same size as pseudorennin and rennin.

The synthesis of pLC7-encoded polypeptide was also analyzed in minicells. Minicells are products of aberrant cell division of strains that carry the _min_ A and _min_ B mutations. The minicells bud off from the parental cell and contain the _E. coli_ transcription/translation system but lack chromosomal DNA. Minicells can be easily fractionated from parental whole cells. When a minicell-producing strain is transformed with plasmid DNA, the minicells will contain plasmid DNA and synthesize plasmid-encoded proteins. Isolated minicells from the minicell producing strain P678-54 transformed with cloned pLC7 DNA produces specific polypeptide which co-electrophoreses with calf prorennin (M.W. = 41,000) and the LC7 polypeptide produced in the in vitro system. When the minicell preparation containing the pLC7-encoded product was fractionated into soluble and membrane compartments, the 41,000 molecular weight radio-labeled polypeptide co-purified with the membrane components.

Whole E. coli cells were also transformed with pLC7 plasmid DNA. The transformed cells were the separated into cytoplasmic, inner membrane and outer membrane fractions Diedrich, D. L., Summers, A. O., and Schnaitman, C. A., J. Bact. 131, 598-607 (1977) as modified by MacGregor, C. H., Bishop, C. W., and Blech, J. E., J. Bact. 137, 574-583 (1979). The pLC7 plasmid encodes a 41,000 M.W. (prorennin size) polypeptide which co-purifies with the outer membrane compartment of the cell. It was not possible to demonstrate acid dependent cleavage of the pLC7 polypeptide while it was associated with the insoluble membrane fraction of the cell.

10. Extraction of Prorennin-Derived
Polypeptide from Insoluble State

From the analyses of fractionated whole cells and minicells, it appeared that the pLC7 polypeptide must be purified away from the insoluble membrane components in order to demonstrate milk clotting activity. In order to extract pLC7 polypeptide activity, cell membranes were treated with a non-ionic detergent to remove contaminating protein components, and the remaining nonsolubilized material, which contained the LC7 polypeptide, was then solubilized in a protein denaturing agent which unfolds most proteins to a random coil structure. The purpose of this treatment is to extract the LC7 polypeptide from E. coli material and to unfold the polypeptide. Production of native LC7 polypeptide requires refolding of the extracted and denatured polypeptide by the removal of the denaturing agent. The pLC7 polypeptide can be further purified by chromatography before or after the denaturing agents are removed from the protein solution.

Example 1

The E. coli strain JM83 (Bethesda Research Laboratories, Inc.) was transformed with pLC7 and grown in L-broth media supplemented with antibiotic. Five grams of JM83/pLC7 cell paste was suspended in buffer containing lysozyme, sonicated, and the mixture was centrifuged. The membrane pellet was suspended in a weight excess of Triton X-100 detergent, and the insoluble material (containing LC7 polypeptide) was collected by centrifugation. This pellet was then solubilized in 6-8M urea. The urea was removed by dialysis in a large volume of buffer lacking urea. This extracted LC7 polypeptide can be acid activated and displays milk clotting activity (Section 11).

Example 2

The membrane fraction was isolated and extracted with 6-8M urea as described in Example 1. This protein solution was then contacted with an ionic chromatographic resin (DEAE-cellulose) and the urea was removed by washing the protein-resin complex. The protein was then eluted from the resin with a salt solution. The soluble protein thus collected contained the LC7 polypeptide which can be acid-activated and will clot milk (Section 11).

Example 3

The membrane fraction containing LC7 polypeptide was solubized in urea and contacted with DEAE-cellulose as in Example 2. LC7 polypeptide was purified by elution of the protein with a gradient of NaCl (0-1M) containing 5-8M urea. Fractions containing the LC7 polypeptide in urea were pooled and the urea was then removed by dialysis. The extracted and purified LC7 polypeptide can be acid activated and contains milk-clotting activity (Section 11).

Following extraction of the LC7 polypeptide in the denaturing agent, several purification procedures were attempted. The presence of LC7 polypeptide was demonstrated by appropriate size of the protein after electrophoresis on polyacrylamide gels (Section 9) and/or milk-clotting activity (Section 11).

Example 4

The membrane fraction of JM83/pLC7 was isolated and extracted with 5-8M urea as described in Example 1. The urea was removed by dialysis. The solution containing LC7 polypeptide was then contacted with an ionic chromatographic resin (DEAE cellulose). The LC7 polypeptide was eluted from the resin with a gradient solution of NaCl (0-1M). The LC7 polypeptide was resolved into two fractions: one containing LC7 polypeptide in a form that can be acid activated and can clot milk and one containing inactive LC7 polypeptide.

Example 5

A solution containing urea extracted and dialysed LC7 polypeptide (see Example 1) was contacted with a hydrophobic chromatographic material; e.g. phenyl-Sepharose (Pharmacia). This material appears to act as an affinity ligand resin. After elution of the

-25-

protein-resin complex with a gradient solution containing decreasing salt and increasing non-ionic detergent concentrations, LC7 polypeptide is resolved into separate fractions containing respectively, polypeptide that could not be activated and polypeptide which could be acid activated.

Purification initiated after the removal of the denaturing agent can resolve the LC7 polypeptide into two forms:  one which is capable of milk clotting activity after activation and one which cannot be activated in this state.  LC7 polypeptide which is remains inactive can be treated to repeated renaturation steps as described in Example 4 and then repurified. Such recycling of LC7 polypeptide allows maximal recovery of active material.

Example 6

A preparation of membrane material containing LC7 polypeptide was extracted with 5-8 M urea and dialyzed. This solution was then contacted with an ionic chromatographic resin (Example 4) and resolved into two fractions:  one which could be acid-activated and one which remained inactive after acidification.  The solution containing the inactive form of LC7 polypeptide was again exposed to 5-8 M urea dialyzed and contacted with the same ionic chromatographic resin.  Upon elution from the resin with a salt gradient, native LC7 polypeptide was recovered in significant yields.

11.  Activation and Assay of Prorennin-Derived Polypeptide

The presence of active LC7 polypeptide was demonstrated by its ability to clot milk following acid or proteolytic treatment.  The milk clot assay is as described by B. Foltmann, Methods in Enzymology, 19, 421-436 (1970).  The assay solution is composed of

-26-

50 mg per ml of nonfat milk powder in water plus 50 mM CaCl , with the pH adjusted to 6.0.  The reaction is carried out at 30°C.  One unit of activity is defined as the amount of enzyme which will clot 1 ml of milk substrate in 100 seconds at 30°C.

Activation of extracted LC7 polypeptide can occur by treatment of this protein with acid or by proteolytic digestion of the LC7 polypeptide with other enzymes. This activation process is then terminated by addition of various chemical agents.  The resultant product of the activation of LC7 polypeptide material can be detected by analysis on polyacrylamide gels or by the clotting of a milk solution in the conventional way.

Example 7

A solution of LC7 polypeptide which had been urea extracted and then dialyzed was acidified to (pH 2 or pH 4.5) with hydrocloric acid for a time sufficient for maximal activation as measured by the increase in milk clotting activity.  The activity of the solution was unaffected by neutralization to pH 5-7 with a basic solution.

Example 8

A solution of LC7 polypeptide prepared as in Example 1 was treated with trypsin, a proteolytic enzyme, in a buffered solution.  The interaction of these two materials continues until maximal activation was achieved, as measured by the increase in milk clotting activity.  The trypsin was then chemically inactivated or physically separated from the activated LC7 polypeptide.

The LC7 prorennin polypeptide is produced in JM83 cells in amounts which constitute at least 1.0% of the total protein of the bacterium, in the range of 10,000 - 20,000 molecules per cell. The level of expression is ten-fold greater than previously published attempts to clone and express a milk-clotting enzyme from rennin mRNA (Alford, B.L. et al., supra). Samples of acid-activated LC7 polypeptide prepared from E. coli have a specific activity greater than 500 units/mg of purified protein. This is greater than 20% of the specific activity of purified calf enzyme.

The LC7 polypeptide was acid activated at pH 4.5 to a rennin size material and its activity compared to commercial rennet samples. The two enzymes were tested by milk clotting assays and found to be indistinguishable with respect to response to temperature (86° - 94°F), milk pH (6.4 - 6.7), milk calcium supplements (0 - .03%), and rate of inactivation by heating 150°F).

The LC7 polypeptide was acid activated at pH 2 to a pseudo-rennin size material and its activity compared to pseudo-rennin derived from purified calf prorennin. By the criteria described above these two enzymatic activities were indistinguishable. These two enzymes also produce similar digestion products upon their addition to a solution of milk or purified caseins.

The recombinant DNA and its host microorganism described herein as JM83/pLC7 was deposited with the American Type Culture Collection, Rockville, Md. and assigned ATCC accession number 39325.

Although the foregoing invention has been described in some detail by way of illustration and example for purposes of clarity of understanding, it will be obvious to one skilled in the art that certain changes and modifications may be practiced within the scope of the appended claims.

## Claims

1.    Recombinant DNA which is capable of directing synthesis of a polypeptide displaying milk clotting activity and which comprises:

a DNA sequence which is capable of autonomous replication in a bacterial host and which further comprises:

(1)    a bacterial promoter sequence; followed by

(2)    a translation start signal; followed by

(3)    amino acid codons; followed by

(4)    a non-bacterial DNA sequence aligned with the translation start signal reading frame and coding for a polypeptide which displays milk clotting activity.

2.    Recombinant DNA in accordance with Claim 1, wherein the polypeptide must be activated in order to display milk clotting activity.

3.    Recombinant DNA in accordance with Claim 2, wherein said polypeptide is activated by exposure to a medium having an acidic pH value.

4.    Recombinant DNA in accordance with Claim 1, wherein said non-bacterial DNA sequence further codes for a precursor peptide sequence which is activated in order to display milk clotting activity.

5. Recombinant DNA in accordance with Claim 1 wherein said amino acid codons comprise:
     (1) a synthetic oligonucleotide,
     (2) a bacterial DNA sequence, or
     (3) a combination of these.

6. Recombinant DNA in accordance with Claim 1, wherein said non-bacterial DNA sequence essentially corresponds to the RNA nucleotide sequence as shown in Figure 2.

7. Recombinant DNA coding for a fusion peptide and comprising the DNA nucleotide sequence corresponding to the RNA nucleotide sequence essentially as shown in Figure 4.

8. The recombinant DNA material contained in E. coli strain JM 83/pLC7 as deposited in the ATCC which is identified as Accession No. 39325.

9. E. coli strain JM 83/pLC7 as deposited in the ATCC which is identified as Accession No. 39325.

10. A polypeptide capable of displaying milk clotting activity comprising the expression product of the recombinant DNA of Claim 1, the polypeptide comprising the amino acid sequence essentially as shown in Figure 2.

11. A polypeptide capable of displaying milk clotting activity comprising the expression product of E. coli strain JM 83/pLC7, ATCC Accession No. 39325 when said strain is cultured.

12. A polypeptide in accordance with Claim 10, further comprising activating said polypeptide by exposure to a medium having a pH value of 2 substantially in order that said polypeptide displays milk clotting activity.

13. A polypeptide in accordance with Claim 10, further comprising activating said polypeptide by exposure to a medium having a pH value of 4.5 substantially in order that said polypeptide displays milk clotting activity.

14. A polypeptide in accordance with Claim 10 further comprising activating said polypeptide by exposure to a medium containing protease in order that said polypeptide displays milk clotting activity.

15. A polypeptide in accordance with Claim 12, 13 or 14, further comprising contacting said polypeptide with a medium containing protein denaturing agent prior to said activation.

16. A method of synthesizing a polypeptide capable of displaying milk clotting activity, which comprises:

(a) isolating a DNA sequence which codes for a polypeptide capable of displaying milk clotting activity;.

(b) constructing recombinant DNA by inserting said DNA sequence into a cloning vector capable of autonomous replication in a bacterial host said cloning vector having a bacterial promoter, translation start signal, and amino acid codons so that said DNA sequence is in reading frame with said translation start signal;

(c) transforming the bacterial host with the recombinant DNA; and

(d) culturing the transformed bacterial host in conditions which promote synthesis of the polypeptide.

17. A method as in Claim 16, further comprising the step of:

extracting the polypeptide from the transformed bacterial host;

18. A method as in Claim 17, further comprising the step of:

purifying the extracted polypeptide by contacting the extracted polypeptide with a medium containing protein denaturing agent and subsequently removing said protein denaturing agent from said medium.

19. A method as in Claim 18, further comprising the step of:

activating the purified polypeptide in a medium containing protease.

20. A method as in Claim 18, further comprising the step of:

activating the polypeptide in a medium having an acidic pH value.

21. A method of clotting milk comprising:
contacting components of milk with
a polypeptide obtained by the process of
Claim 16.

22. A method as in Claim 16, further comprising the step of:

extracting the polypeptide from an insoluble state.

23. A method as in Claim 22, further comprising the step of:

reextracting the polypeptide which remains in an inactive state.

Fig. I.

P = Pst
B = Bam HI
T = Taq

Translation of 5G3

```
                                        -16                    -10
                                        met arg cys leu val val leu leu ala
CGGCUGGACCCAGAUCCAAG                    AUG AGG UGU CUC GUG GUG CUA CUU GCU


                                          1
val phe ala leu ser gln gly ala glu ile thr arg ile pro leu
GUC UUC GCU CUC UCC CAG GGC GCU GAG AUC ACC AGG AUC CCU CUG


      10                                                20
tyr lys gly lys ser leu arg lys ala leu lys glu his gly leu
UAC AAA GGC AAG UCU CUG AGG AAG GCG CUG AAG GAG CAU GGG CUU


                            30
leu glu asp phe leu gln lys gln gln tyr gly ile ser ser lys
CUG GAG GAC UUC CUG CAG AAA CAG CAG UAU GGC AUC AGC AGC AAG


      40                                          50
tyr ser gly phe gly glu val ala ser val pro leu thr asn tyr
UAC UCC GGC UUC GGG GAG GUG GCC AGC GUG CCC CUG ACC AAC UAC


                            60
leu asp ser gln tyr phe gly lys ile tyr leu gly thr pro pro
CUG GAU AGU CAG UAC UUU GGG AAG AUC UAC CUC GGG ACC CCG CCC


      70                                          80
gln glu phe thr val leu phe asp thr gly ser ser asp phe trp
CAG GAG UUC ACC GUG CUG UUU GAC ACU GGC UCC UCU GAC UUC UGG


                            90
val pro ser ile tyr cys lys ser asn ala cys lys asn his gln
GUA CCC UCU AUC UAC UGC AAG AGC AAU GCC UGC AAA AAC CAC CAG


      100                                         110
arg phe asp pro arg lys ser ser thr phe gln asn leu gly lys
CGC UUC GAC CCG AGA AAG UCG UCC ACC UUC CAG AAC CUG GGC AAG


                            120
pro leu ser ile his tyr gly thr gly ser met gln gly ile leu
CCC CUG UCU AUC CAC UAC GGG ACA GGC AGC AUG CAG GGC AUC CUA


      130                                         140
gly tyr asp thr val thr val ser asn ile val asp ile gln glu
GGC UAU GAC ACC GUC ACU GUC UCC AAC AUU GUG GAC AUC CAG GAG


                            150
thr val gly leu ser thr gln glu pro gly asp val phe thr tyr
ACA GUA GGC CUG AGC ACC CAG GAG CCC GGG GAC GUC UUC ACC UAU
```

Fig. 2 (A)

```
160                                                    170
ala glu phe asp gly ile leu gly met ala tyr pro ser leu ala
GCC GAA UUC GAC GGG AUC CUG GGG AUG GCC UAC CCC UCG CUC GCC


                              180
ser glu tyr ser ile pro val phe asp asn met met asn arg his
UCA GAG UAC UCG AUA CCC GUG UUU GAC AAC AUG AUG AAC AGG CAC


   190                                                 200
leu val ala gln asp leu phe ser val tyr met asp arg asn gly
CUG GUG GCC CAA GAC CUG UUC UCG GUU UAC AUG GAC AGG AAU GGC


                              210
gln glu ser met leu thr leu gly ala ile asp pro ser tyr tyr
CAG GAG AGC AUG CUC ACG CUG GGG GCC AUC GAC CCG UCC UAC UAC


   220                                                 230
thr gly ser leu his trp val pro val thr val gln gln tyr trp
ACA GGG UCC CUG CAC UGG GUG CCC GUG ACA GUG CAG CAG UAC UGG


                              240
gln phe thr val asp ser val thr ile ser gly val val val ala
CAG UUC ACU GUG GAC AGU GUC ACC AUC AGC GGU GUG GUU GUG GCC


   250                                                 260
cys glu gly gly cys gln ala ile leu asp thr gly thr ser lys
UGU GAG GGU GGC UGU CAG GCC AUC CUG GAC ACG GGC ACC UCC AAG


                              270
leu val gly pro ser ser asp ile leu asn ile gln gln ala ile
CUG GUC GGG CCC AGC AGC GAC AUC CUC AAC AUC CAG CAG GCC AUU


   280                                                 290
gly ala thr gln asn gln tyr asp glu phe asp ile asp cys asp
GGA GCC ACA CAG AAC CAG UAC GAU GAG UUU GAC AUC GAC UGC GAC


                              300
asn leu ser tyr met pro thr val val phe glu ile asn gly lys
AAC CUG AGC UAC AUG CCC ACU GUG GUC UUU GAG AUC AAU GGC AAA


   310                                                 320
met tyr pro leu thr pro ser ala tyr thr ser gln asp gln gly
AUG UAC CCA CUG ACC CCC UCC GCC UAU ACC AGC CAA GAC CAG GGC


                              330
phe cys thr ser gly phe gln ser glu asn his ser gln lys trp
UUC UGU ACC AGU GGC UUC CAG AGU GAA AAU CAU UCC CAG AAA UGG


   340                                                 350
ile leu gly asp val phe ile arg glu tyr tyr ser val phe asp
AUC CUG GGG GAU GUU UUC AUC CGA GAG UAU UAC AGC GUC UUU GAC


                              360                    365
arg ala asn asn leu val gly leu ala lys ala ile OP
AGG GCC AAC AAC CUC GUG GGG CUG GCC AAA GCC AUC UGA UCACACCCC
```

Fig. 2(B)

Fig. 3a.

Fig. 3 b.

p LC 2

Cla I    Pst

Fig. 3 c.

p LC 2

Cla I    Pst

+

Pst

Pst          Pst

5 G

Cla I    Pst

Pst

p LC 7

5/6

0123928

```
                                              met thr met ile thr asn
AAUUGUGAGCGGAUAACAAUUUCACACAGGAAACAGCU  AUG ACC AUG AUU ACG AAU
                    6                  10
ser arg gly ser ile pro leu tyr lys gly lys ser leu arg lys ala
UCC CGG GGA UCG AUC CCU CUG UAC AAA GGC AAG UCU CUG AGG AAG GCG
            20                                          30
leu lys glu his gly leu leu glu asp phe leu gln lys gln gln tyr
CUG AAG GAG CAU GGG CUU CUG GAG GAC UUC CUG CAG AAA CAG CAG UAU
                            40
gly ile ser ser lys tyr ser gly phe
GGC AUC AGC AGC AAG UAC UCC GGC UUC
```